# EUROPÄISCHE PATENTANMELDUNG

(11) **EP 2 168 487 A1**
(43) Veröffentlichungstag der Anmeldung: **31.03.2010**
(21) Anmeldenummer: 09154900.6
(22) Anmeldetag: 11.03.2009
(51) Int. Cl.: A61B 6/00, A61B 6/06, G21K 1/04, G01K 11/30, G01K 15/00, G01K 13/00, G01K 1/02, A61B 6/10, A61B 5/00, A61B 18/00, A61F 7/00, A61B 19/00, A61N 7/02, A61B 17/00

(54) **Verfahren und Vorrichtung zur thermischen Brusttumor-Behandlung mit 3D Monitorfunktion**

(30) Priorität: 29.09.2008 DE 102008042430
(71) Anmelder: MIR Medical Imaging Research Holding GmbH, 91096 Möhrendorf (DE)
(72) Erfinder: Kalender, Willi, 91096, Möhrendorf (DE); Schilling, Harry, 85072, Eichstätt (DE)
(74) Vertreter: Lohr, Georg

(57) **Zusammenfassung**

Ein Röntgengerät zur Überwachung einer thermischen Behandlung von menschlichem Gewebe fertigt vor Beginn der Behandlung zuerst eine Referenzaufnahme des zu behandelnden Gewebes an. Danach wird eine thermische Behandlung durchgeführt. Während der Behandlung oder auch in Behandlungspausen werden durch das Röntgengerät Kontrollaufnahmen angefertigt. Diese Kontrollaufnahmen werden als Partialvolumenaufnahmen mit reduzierter Strahlenbelastung durchgeführt. Durch Vergleich der Kontrollaufnahmen mit der Referenzaufnahme kann auf die Änderung der Gewebetemperatur und auch der Gewebeeigenschaften geschlossen werden.

## Beschreibung

### Technisches Gebiet

Die Erfindung betrifft ein Röntgengerät sowie ein Verfahren zur Überwachung einer Diathermie- Behandlung. Die Erfindung betrifft insbesondere ein Röntgengerät zur Abbildung der weiblichen Brust (Mammografie) sowie ein Verfahren zur Überwachung einer diathermischen Brusttumor- Behandlung.

### Stand der Technik

Zur Untersuchung der weiblichen Brust sind verschiedene Röntgengeräte bekannt. Unter einer Liege, auf der eine zu untersuchende Patientin liegt, befindet sich eine Röntgenvorrichtung mit einer rotierenden Gantry, welche eine Röntgenröhre und einem Detektor aufweist. Ein solches Gerät ist beispielsweise in der US 4,015,836 offenbart. Mit derartigen Röntgengeräten ist eine Diagnose von Erkrankungen der Brust, insbesondere von Tumoren in der Brust möglich. Diese Tumore werden auf verschiedene Arten behandelt. Ein bewährtes Verfahren ist die Hyperthermie. Hierbei wird das Gewebe im Bereich des Tumors so stark erhitzt, dass die Zellen des Tumors absterben. Ein solches Hyperthermiegerät ist in der US 6,358,246 offenbart. Zur Behandlung des Tumors wird eine Elektrode in den Tumor eingebracht. Durch diese wird hochfrequenter Strom in das Gewebe des Tumors geleitet. Als Maß für die maximale Leistung wird hier ein Anstieg der elektrischen Impedanz des Gewebes herangezogen. Problematisch hierbei ist, dass die Erwärmung nicht gezielt gesteuert werden kann, denn die Temperatur ist keine direkte Funktion des Stromflusses oder der in das Gewebe eingebrachten Energie. Eine kontrollierte Therapie des Tumors ist mit einem solchen Instrument nur schwer möglich.

In der US 6,684,097 B1 ist ein Verfahren zur Temperaturüberwachung während einer diathermischen Behandlung einer Brust offenbart. Dieses Verfahren fertigt vor der Behandlung eine hochauflösende dreidimensionale Röntgenaufnahme von der Brust an und speichert diese ab. Während der Behandlung werden weitere hochauflösende dreidimensionale Aufnahmen von der Brust angefertigt und mit der ursprünglichen Aufnahme verglichen. Aus einer Differenz der einzelnen Pixelwerte wird auf eine Temperaturänderung geschlossen. Nachteilig an diesem Verfahren ist die hohe Strahlenbelastung durch die hochauflösenden dreidimensionalen Aufnahmen.

### Darstellung der Erfindung

Der Erfindung liegt die Aufgabe zugrunde, ein medizinisches Gerät vorzustellen, mit dem eine thermische Tumorbehandlung, insbesondere eine diathermische Tumorbehandlung einer weiblichen Brust kontrolliert erfolgen kann, wobei die Strahlenbelastung gegenüber dem Stand der Technik wesentlich reduziert wird. Ein weiterer Aspekt der Erfindung ist ein Verfahren zur Überwachung und/oder Steuerung eines medizinischen Gerätes zur Überwachung beziehungsweise zu kontrollierten diathermischen Tumorbehandlung, insbesondere einer weiblichen Brust, wobei die Strahlenbelastung gegenüber dem Stand der Technik wesentlich reduziert wird.

Diese Aufgabe wird durch ein Röntgengerät nach Anspruch 1 gelöst. Vorteilhafte Ausgestaltungen der Erfindung sind in den Unteransprüchen angegeben.

Ein erfindungsgemäßes Verfahren umfasst die folgenden Schritte:
a. Erstellen einer Röntgenaufnahme von der zu behandelnden Region; Hierbei wird zuerst wenigstens eine Referenzaufnahme des zu behandelnden Gewebes erstellt. Grundsätzlich kann eine Vielzahl von verschiedenen Referenzaufnahmen erstellt werden.
b. Thermische Behandlung;
   Nach der Referenzaufnahme erfolgt eine diathermische Behandlung durch Erwärmen des Gewebes. Es kann eine neue Behandlung begonnen oder auch eine vorhergehende Behandlung fortgesetzt werden. Zur Erwärmung wird Energie, vorzugsweise als elektrische Energie, aber auch durch Bestrahlung in das Gewebe eingekoppelt.
c. Erstellen von Kontrollaufnahmen;
   Zur Kontrolle der diathermischen Behandlung werden vorzugsweise in vorgegebenen Intervallen Röntgenaufnahmen angefertigt. Diese werden mit wenigstens einer der in Schritt a. angefertigten Referenzaufnahmen verglichen. Im einfachsten Falle erfolgt eine einfache Subtraktion der Helligkeitswerte. Aufgrund der Differenz zur Referenzaufnahme kann auf die Temperaturänderung des Gewebes geschlossen werden. Damit kann die Funktion des Diathermiegerätes überwacht werden. Kontrollaufnahmen können grundsätzlich während der Behandlung oder auch in kurzen Behandlungspausen durchgeführt werden. Erfindungsgemäß erfolgen diese Kontrollaufnahmen mit einer insgesamt niedrigeren Strahlendosis als eine normale hochauflösende dreidimensionale Aufnahme. Weiterhin wird diese Aufnahme als Teilvolumenaufnahme realisiert, wobei nur ein bestimmter für die Temperaturüberwachung ausgewählter Bereich bestrahlt und aufgenommen wird.

Röntgenuntersuchungen haben gezeigt, dass sich bei Diathermiebehandlungen, d.h. bei einer Temperaturänderung des Gewebes die Röntgeneigenschaften um einige Hounsfield Units ändern. Der zu Grunde liegende physikalische Effekt ist vermutlich eine Dichteänderung des Gewebes.

Durch ein erfindungsgemäßes Verfahren ist es nun möglich, ein Temperaturprofil der thermisch behandelten Region zu erstellen. Durch den Einsatz von 3 D-Röntgengeräten kann auch ein räumliches Temperaturprofil erstellt werden. Aufgrund des Temperaturprofils kann der Verlauf der Behandlung präzise kontrolliert werden. Entsprechend kann das die Temperaturänderung erzeugende Gerät entsprechend eingestellt und/oder geregelt werden. Ebenso kann bei Erreichen eines bestimmten Temperaturprofils auch die Behandlung beendet werden. Bei einem erfindungsgemäßen Verfahren kann die Strahlendosis relativ klein gehalten werden, da nur eine Teilvolumenaufnahme des erwärmten Bereichs erstellt werden muss. Weiterhin kann durch das Röntgengerät bereits vor Beginn der Behandlung und auch während der Behandlung das die Temperaturänderung erzeugende Gerät oder eine Sonde des Gerätes in dem Gewebe präzise positioniert werden.

Bevorzugt rotiert die Gantry während der thermischen Behandlung kontinuierlich und fertigt in vorgegebenen Zeitabständen Messungen an. Bevorzugt umfasst eine Messung zwei Aufnahmen, die um 90 Grad gegeneinander versetzt sind. Selbstverständlich wird nur zur Durchführung der Messungen beziehungsweise Abbildungen die Röntgenröhre aktiviert. Da es hier genügt, die Messungen in relativ großen Zeitintervallen, beispielsweise 1, 5, 10, 20 oder 60 Sekunden durchzuführen, kann die Gantry relativ langsam rotieren. Bei den größeren Zeitintervallen wie 10, 20 oder 60 Sekunden, rotiert die Gantry bevorzugt mit einer Geschwindigkeit, die diese mehrere Umdrehungen innerhalb dieser Zeitintervallen durchführen lässt, so dass der zeitliche Versatz zwischen zwei um 90 Grad gegeneinander versetzten Messungen reduziert wird. Beispielhaft wird bei Messintervallen von 60 Sekunden eine Umdrehung pro Sekunde durchgeführt.

Damit ist der Zeitversatz zwischen zwei um 90 Grad gegeneinander versetzten Messungen 0,25 Sekunden.

Bevorzugt ist eine Blende 56 vorgesehen, die derart eingestellt wird, dass die Röntgenstrahlung des Röntgengerätes nur einen vorgegebenen Bereich erfasst. Diese Blende wird bei den einzelnen Messungen abhängig von der Position der Gantry eingestellt.

Alternativ zu den um 90 Grad versetzten Aufnahmen können auch mehrere Aufnahmen aus verschiedenen Positionen der Gantry 10 durchgeführt werden. Hierbei wird die Blende 56 für jede dieser Positionen derart eingestellt, dass die Röntgenstrahlung des Röntgengerätes nur einen vorgegebenen Bereich erfasst.

Besonders günstig ist es, wenn zum Erstellen der wenigstens einen Kontrollaufnahme mehrere Aufnahmen während einer Kreis- oder Spiralbewegung einer Gantry 10 durchgeführt werden und eine Blende 56 entsprechend der Bewegung derart eingestellt wird, dass die Röntgenstrahlung des Röntgengerätes nur einen vorgegebenen Bereich erfasst. Hierbei kann die Blende wahlweise in Schritten entsprechend der Bewegung oder auch stufenlos nachgeführt werden.

Ein erfindungsgemäßes Röntgengerät umfasst eine Auflagefläche 20 für einen Patienten 30 sowie eine Gantry 10 mit einer Röntgenröhre und einem Detektor, welche durch einen Z-Achsen-Antrieb gegenüber dem Patienten verschoben werden kann. Weiterhin bietet das Röntgengerät einen Zugang für ein Gerät, welches das zu behandelnde Gewebe erwärmt und/oder abkühlt. Die Erwärmung bzw. Abkühlung des Gewebes kann durch
- elektrischen Strom, vorzugsweise hochfrequenten Strom,
- fokussierten Ultraschall,
- Wärmestrahlung, bevorzugt im fernen Infrarotbereich,
- Terahertz-Strahlung,
- Ein Wärme und/oder Kühlmedium wie eine Flüssigkeit oder ein Gas, bevorzugt ein flüssiges Gas, besonders bevorzugt flüssiger Stickstoff
oder auch durch eine Kombination davon erfolgen.

Weiterhin weist ein erfindungsgemäßes Röntgengerät Mittel zur Kollimierung der Röntgenstrahlung der Röntgenröhre auf. Um Aufnahmen aus mehreren Positionen anfertigen zu können, ist es notwendig, die Strahlkollimierung positionsabhängig einzustellen. Dies erfolgt mittels einer Blende 56. Diese Blende ermöglicht bevorzugt eine Einengung des Strahlenfächer 16 in einer Ebene senkrecht zur Rotationsachse 12. Optional ist auch noch eine Einengung in einer Ebene parallel zur Rotationsachse möglich.

Besonders günstig ist es, wenn Methoden und Verfahren zur Verbesserung der zeitlichen und/oder räumlichen Auflösung bei der Kombination der verschiedenen Aufnahmen genutzt werden. Da sich die Aufnahmen nur innerhalb lokaler Parameter, wie z.B. die Temperatur in einem bestimmten zu therapierenden Subvolumen unterscheiden verbessert die zeitlich/räumliche Verknüpfung der Aufnahmen die Bildqualität bzw. minimiert bei gleichbleibender Bildqualität die dem Patienten zu applizierende Strahlendosis. Besonders vorteilhaft sind Verfahren, die unter dem Namen PICCS (Prior image constrained compressed sensing) und HYPR (HighlY constrained backPRojection) bekannt sind.

In einer weiteren Ausführung von der Erfindung ist wenigstens ein Behälter mit einem Referenzmedium beziehungsweise einer Referenzflüssigkeit 161 vorgesehen. Eine solche Referenzflüssigkeit kann beispielsweise Wasser oder Glycerin sein. Zur exakten Temperaturüberwachung kann ein Temperatursensor 162 dienen. Da die Änderungen der Röntgeneigenschaften des Gewebes durch Temperaturänderungen nur relativ gering sind, kann jeweils durch die Referenzflüssigkeit oder durch ein Referenzmedium im allgemeinen Falle eine Kalibrierung des Röntgengerätes erfolgen. Bevorzugt erfolgt die Kalibrierung beziehungsweise die Messungen des Referenzmediums vor und/oder während einer Referenzaufnahme und/oder einer Kontrollaufnahme.

Ein weiterer Gegenstand der Erfindung ist ein Röntgengerät zur Durchführung der zuvor beschriebenen Verfahren. Dieses Röntgengerät ist bevorzugt ein CT-Scanner und weist eine verstellbare Blende 56 auf. Bevorzugterweise ist auch noch wenigstens ein Referenzmedium 161 vorgesehen.

Ein erfindungsgemäßes medizinisches System zur kontrollierten diathermischen Behandlung von Körpergewebe umfasst ein Röntgengerät wie zuvor dargestellt, sowie ein Gerät zur Erwärmung und/oder Abkühlung von Körpergewebe. Es kann sich hierbei beispielsweise um ein Diathermiegerät oder auch um ein Gerät zur kryotherapeutischen Behandlung handeln.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt ein erfindungsgemäßes Röntgengerät zur Untersuchung bzw. Behandlung einer weiblichen Brust
Figur 2 zeigt die Funktion der dynamischen Kollimierung
Figur 3 zeigt ein erfindungsgemäßes Röntgengerät im seitlichen Schnitt

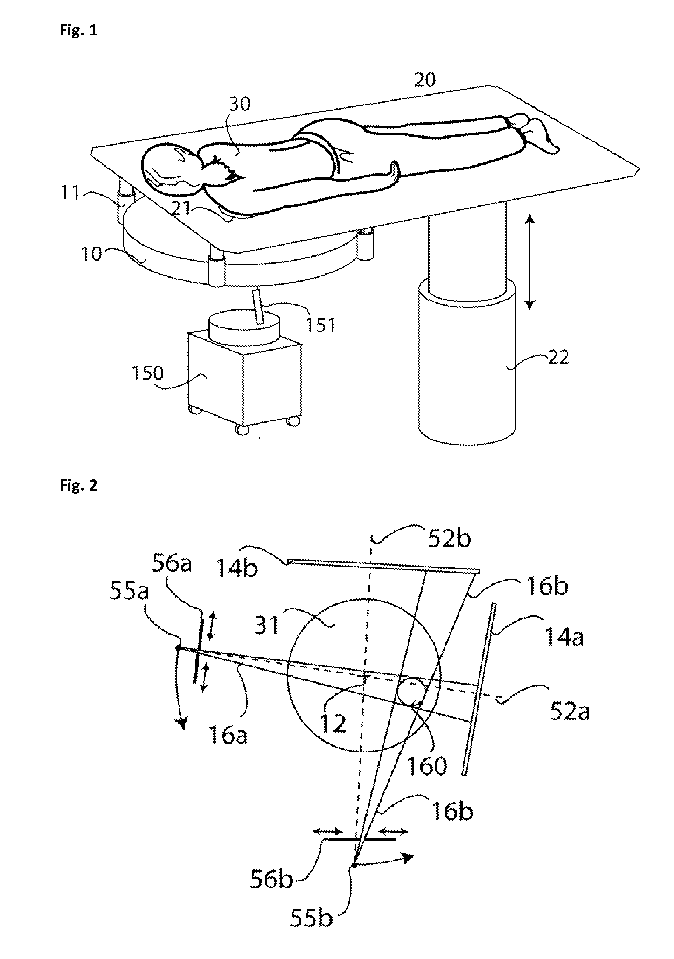

In Figur 1 ist ein erfindungsgemäßes Röntgengerät zur Untersuchung bzw. Behandlung einer weiblichen Brust dargestellt. Auf der Auflagefläche 20 liegt eine Patientin 30. Die zu untersuchende Brust hängt über einen Brustausschnitt 21 durch die Patientenliege 20 in den Aufnahmebereich einer Gantry 10. Die Gantry 10 ist eine Spiral-Computertomographen Gantry mit einer Röntgenröhre und einem Detektor, welche sich um die zu untersuchende Brust drehen. Während der Drehung wird die Brust abgebildet. Gleichzeitig mit der Drehung wird über den Gantryhubantrieb 11 eine Verschiebung in vertikaler Richtung durchgeführt, so dass die Brust spiralförmig abgetastet wird. Die Patientenliege 20 ist über einen Patientenliegenhubantrieb 22 in der Höhe verstellbar. Das Diathermiegerät 150 versorgt das Instrument 151 mit Energie, welche zu einer Erwärmung der betreffenden Region in der Brust der Patientin führt. In diesem Beispiel ist das Erfindungsgemäße Gerät mit einer horizontal angeordneten Auflagefläche für den Patienten dargestellt. Grundsätzlich ist aber auch eine senkrecht stehende oder in anderen Winkeln angeordnete Auflagefläche möglich.

In Figur 2 ist die Funktion der der dynamischen Fokussierung zur Minimierung der Strahlungsbelastung während der Temperaturmessung dargestellt. Um die Strahlungsbelastung möglichst gering zu halten wird während der Temperaturmessung kein vollständiger, hochauflösender 3-D-Scan vorgenommen. Vielmehr wird hier beispielhaft jeweils eine Abbildung aus zwei um 90 Grad zueinander versetzten Positionen vorgenommen. Es handelt sich hierbei um dieselben Komponenten. Entsprechend der Position ist hinter dem Bezugszeichen der entsprechende Buchstabe a für die erste Position und der Buchstabe b für die zweite Position angegeben. Eine solche Anordnung reicht in der Regel aus, um mit hinreichender Genauigkeit die Temperaturverteilung zu ermitteln. Diese basiert auch auf der Erkenntnis, dass sich in näherungsweise homogenen Materialien wie dem menschlichen Körpergewebe eine in erster Näherung kugelförmige Temperaturverteilung entwickelt. Eine erste Aufnahme erfolgt hier mit Röntgenstrahlung ausgehend von einer Position der Röntgenröhre mit einem ersten Fokuspunkt 55a. Durch eine zu dieser Position entsprechend eingestellte Blende 56a wird ein Strahlenfächer 16a erzeugt, welcher gerade so groß ist, dass der zu untersuchende Bereich ROI 160 abgedeckt wird und möglichst wenig Strahlung in die Nachbarschaft abgegeben wird. Die Strahlung wird mit dem Detektor in der Position 14a aufgefangen und ausgewertet. Es ist noch der zu dieser Position der zugehörige Zentralstrahl 52a dargestellt. In einer weiteren Position der Röntgenröhre ergibt sich die Position des Fokuspunkt 55b. Hier wird durch eine entsprechend angepasste Einstellung der Blende 56b ein Strahlenfächer 16b erzeugt, der nun wiederum möglichst genau den zu untersuchenden Bereich ROI 160 umfasst. Die Strahlung wird durch den Detektor in der Position 14b erfasst und ausgewertet. Der zugehörige Zentralstrahl 52b liegt die außerhalb des Strahlenfächers 16b.

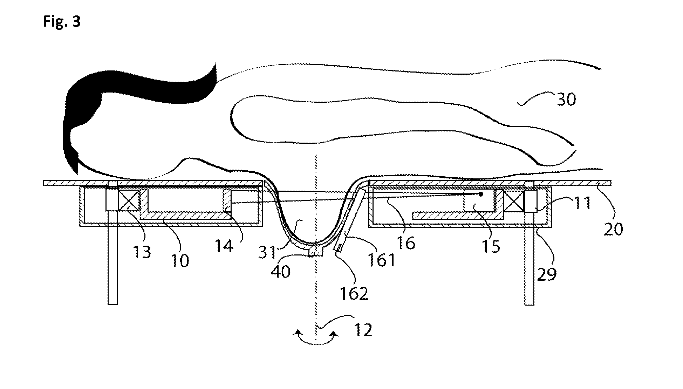

In Figur 3 ist eine erfindungsgemäße Vorrichtung im Schnitt dargestellt. Innerhalb des Gantrygehäuses 29 befindet sich eine Röntgenröhre 15, welche einen Strahlenfächer 16 zur Durchstrahlung der in einer Fixiervorrichtung 40 fixierten Brust 31 erzeugt. Die Strahlung wird von einem Detektor 14 empfangen und zu einer Auswerteeinheit (hier nicht dargestellt) geführt. Die Gantry ist einerseits mit einem Gantrydrehlager 13 um die Brust mit der Drehachse 12 drehbar und über den Gantryhubantrieb 11 in ihrer Höhe beziehungsweise im Abstand zur Patientin verschiebbar. Damit ist eine spiralförmige Abtastung der zu untersuchenden Brust 31 möglich. Das hier dargestellte Röntgengerät erlaubt eine Durchführung von Kontrollaufnahmen während der Behandlung, da das Diathermiegerät durch die Gantry hindurchgreift und diese bei ihrer Spiralbewegung während einer Aufnahme nicht behindert. Mit dem Behälter mit einem Referenzmedium 161 ist eine Kalibrierung der Vorrichtung möglich. Die exakte Temperatur der Referenzflüssigkeit wird durch den Temperatursensor 162 ermittelt. Optional sind noch Mittel zum Beheizen und/oder Abkühlen der Referenzflüssigkeit vorhanden.

### Bezugszeichenliste

- 10: Gantry
- 11: Z-Achsen-Antrieb
- 12: Rotationsachse
- 13: Gantrydrehlager
- 14: Detektor
- 15: Röntgenröhre
- 16: Strahlenfächer
- 20: Auflagefläche
- 21: Brustausschnitt
- 22: Patientenliegenhubantrieb
- 29: Gantrygehäuse
- 30: Patient
- 31: Brust
- 40: Fixiervorrichtung
- 52: Zentralstrahl
- 55: Fokuspunkt (Brennfleck)
- 56: Blende
- 150: Diathermiegerät
- 151: Gerät zur thermischen Behandlung
- 160: ROI Region Of Interest
- 161: Referenzmedium
- 162: Temperatursensor

## Patentansprüche

1. Verfahren zur Überwachung eines Gerätes zur thermischen Behandlung von Körpergewebe einer bestimmten Region eines menschlichen Körpers umfassend die Schritte:
a. Erstellen wenigstens einer dreidimensionalen Referenzaufnahme von der zu behandelnden Region durch ein Röntgengerät;
b. Durchführen der thermischen Behandlung;
c. Erstellen von wenigstens einer Kontrollaufnahme durch das Röntgengerät;
d. Erstellen eines Temperaturänderungsprofils durch Vergleich der wenigstens einen Kontrollaufnahme mit der wenigstens einen Referenzaufnahme,
**dadurch gekennzeichnet, dass**
zum Erstellen der wenigstens einen Kontrollaufnahme diese als eine Teilvolumenaufnahme der zu behandelnden Region mit niedrigerer Strahlendosis als eine normale dreidimensionale Röntgenaufnahme durchgeführt wird

2. Verfahren nach Anspruch 1,
**dadurch gekennzeichnet, dass**
vor dem Erstellen der wenigstens einen Kontrollaufnahme eine Blende (56) derart eingestellt wird, dass die Röntgenstrahlung des Röntgengerätes nur einen vorgegebenen Bereich erfasst.

3. Verfahren nach Anspruch 1 oder 2,
**dadurch gekennzeichnet, dass**
zum Erstellen der wenigstens einen Kontrollaufnahme zwei Aufnahmen aus zwei um 90 Grad gegeneinander versetzten Positionen einer Gantry (10) mit Röntgenröhre (15) und Röntgendetektor (14) durchgeführt werden.

4. Verfahren nach Anspruch 2,
**dadurch gekennzeichnet, dass**
zum Erstellen der wenigstens einen Kontrollaufnahme zwei Aufnahmen aus zwei um 90 Grad gegeneinander versetzten Positionen einer Gantry (10) mit Röntgenröhre (15) und Röntgendetektor (14) durchgeführt werden und eine Blende (56) für jede dieser Positionen derart eingestellt wird, dass die Röntgenstrahlung des Röntgengerätes nur einen vorgegebenen Bereich erfasst.

5. Verfahren nach Anspruch 2,
**dadurch gekennzeichnet, dass**
zum Erstellen der wenigstens einen Kontrollaufnahme mehrere Aufnahmen aus verschiedenen Positionen einer Gantry (10) mit Röntgenröhre (15) und Röntgendetektor (14) durchgeführt werden und eine Blende (56) für jede dieser Positionen derart eingestellt wird, dass die Röntgenstrahlung des Röntgengerätes nur einen vorgegebenen Bereich erfasst.

6. Verfahren nach Anspruch 2,
**dadurch gekennzeichnet, dass**
zum Erstellen der wenigstens einen Kontrollaufnahme mehrere Aufnahmen während einer Kreis- oder Spiralbewegung einer Gantry (10) mit Röntgenröhre (15) und Röntgendetektor (14) durchgeführt werden und eine Blende (56) entsprechend der Bewegung derart eingestellt wird, dass die Röntgenstrahlung des Röntgengerätes nur einen vorgegebenen Bereich erfasst.

7. Verfahren nach einem der vorhergehenden Ansprüche,
**dadurch gekennzeichnet, dass**
Verfahren zur Verbesserung der zeitlichen und/oder räumlichen Auflösung wie Namen PICCS (Prior image constrained compressed sensing) und HYPR (HighlY constrained backPRojection) eingesetzt werden.

8. Verfahren nach Anspruch 1,
**dadurch gekennzeichnet, dass**
das Röntgengerät wahlweise vor oder gleichzeitig mit der Durchführung einer Referenzaufnahme oder einer Kontrollaufnahme durch Vergleich mit wenigstens einem Referenzmedium 161 kalibriert wird.

9. Verfahren nach Anspruch 8,
**dadurch gekennzeichnet, dass**
mit der Durchführung einer Referenzaufnahme oder einer Kontrollaufnahme auch die Aufnahme wenigstens eines Referenzmediums 161 durchgeführt wird.

10. Verfahren nach Anspruch 8 oder 9,
**dadurch gekennzeichnet, dass**
gleichzeitig die Temperatur des Referenzmediums gemessen wird.

11. Röntgengerät zur Durchführung des Verfahrens nach Anspruch 1.

12. Röntgengerät nach Anspruch 11,
**dadurch gekennzeichnet, dass**
das Röntgengerät ein CT-Scanner ist.

13. Röntgengerät nach Anspruch 12,
**dadurch gekennzeichnet, dass**
das Röntgengerät eine verstellbare Blende (56) aufweist.

14. Röntgengerät nach Anspruch 13,
**dadurch gekennzeichnet, dass**
ein Referenzmedium (161) zur Kalibrierung vorgesehen ist.

15. Medizinisches System zur kontrollierten thermischen Behandlung von Körpergewebe,
umfassend einen Röntgengerät nach einem der Ansprüche 10 bis 13 sowie ein Gerät zur Erwärmung und/oder Abkühlung von Körpergewebe einer bestimmten Region.
